# EUROPEAN PATENT SPECIFICATION

(11) **EP 1 087 742 B1**
(45) Date of publication and mention of the grant of the patent: **31.03.2004**
(21) Application number: 99954342.4
(22) Date of filing: 19.10.1999
(51) Int. Cl.: A61F 13/15, B23P 15/24

(54) **FILM MADE OF HOLED PLASTIC MATERIAL THREE-DIMENSIONALLY SHAPED AND RELATED MATRIX FOR ITS REALISATION**
DREIDIMENSIONALE, PERFORIERTE PLASTIKFOLIE SOWIE MATRIZE ZUR HERSTELLUNG DERSELBEN
FILM FAIT D'UNE MATIERE PLASTIQUE PERCE EN FORME TRIDIMENSIONNELLE ET MATRICE CORRESPONDANTE PERMETTANT DE REALISER CE FILM

(30) Priority: 15.04.1999 IT RM990229
(43) Date of publication of application: 04.04.2001
(73) Proprietor: Tredegar Film Products Italia S.r.l, 66020 S.Giovanni Teatino (IT)
(72) Inventor: IULIANETTI, Lino, Torre dei Passeri (IT)
(74) Representative: Lanzoni, Luciano
(86) International application number: PCT/IT1999/000330
(87) International publication number: WO 2000/062729

(56) References cited:
- EP-A- 0 138 601
- WO-A-90/12481
- WO-A-97/22434
- DE-A- 2 746 440
- DE-A- 2 948 376
- US-A- 3 293 737
- US-A- 4 535 020
- US-A- 4 601 868
- US-A- 4 604 156
- US-A- 4 780 352
- US-A- 5 766 441

## Description

The present invention relates to a film made of holed plastic material three-dimensionally shaped and a related matrix for its realisation. A film of this kind can be employed as a filtering layer in different sectors like that of hygienic-sanitary items, such as ladies' sanitary napkins, nappies for children and pads for incontinence protection. It can be used as a filtering layer or film for packing meats destined for consumption, allowing for their better preservation and longer duration. This film can also be used in agriculture for mulching or protecting crops on the soil or as a protective wrapping for fruit on the plant while they ripen. for instance grapes.

Below, as a foreword, are listed the definitions of some parameters used in the manufacture of holed plastic material.

The term "liquid strike-through time" indicates the time taken by a known quantity of known liquid to traverse a given filtering layer or film, and thus determines its ability to be traversed by that liquid. The measurement is performed following procedures in accordance with the current Edana 150.3 standard.

"Coverstock wetback" is the parameter that measures the ability of a filtering layer or film to oppose the back-flow of a known liquid in the direction of the skin as soon as it penetrates into the layer, and the measurement is performed following procedures in accordance with the current Edana 151.1 standard.

The term "gloss" refers to the ratio between the light specularly reflected by a surface, and total reflected light. Hence in defining the superficial gloss of a film, only the light reflected with an angle equal to the incidence angle is taken into consideration, and not light reflected in all other directions.

### Background Art

For many years, after US Patent 3,929,135 by Procter & Gamble (1974), efforts have been mainly focused on making holed plastic material films as similar to tissues as possible, with fibre-like elements defining openings which in turn are divided into smaller openings or polygonal shapes with such holes as to confer the appearance of twisted fibres or obtaining small asperities on the surface of the films of plastic material in order to diminish their gloss, for example, see US-A-4 780 352, DE 2 948 376.

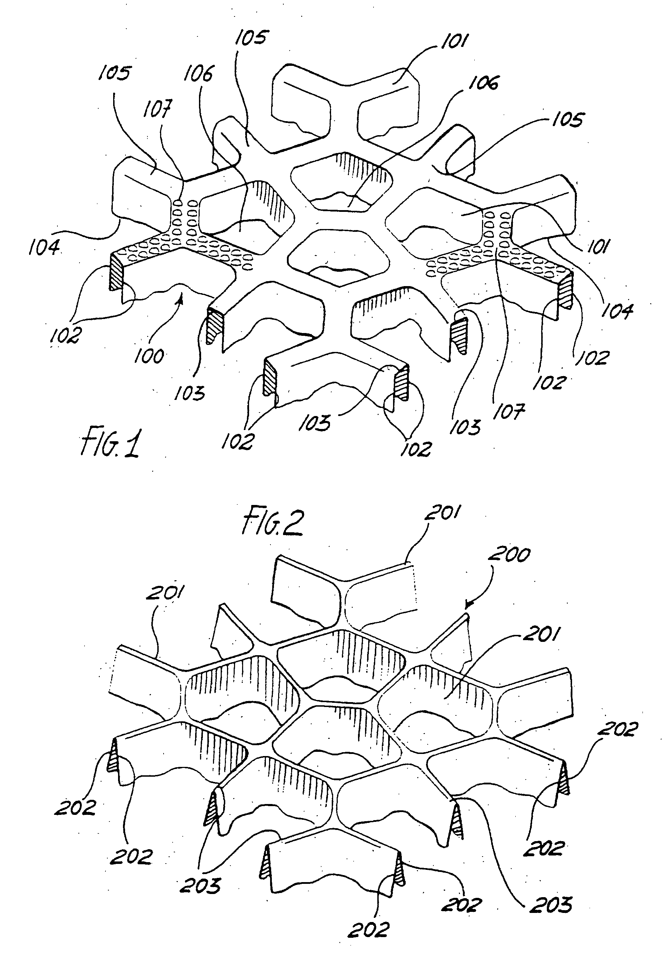

Figure 1 shows an enlarged schematic view of a portion of a first film made of holed plastic material 100, known in the prior art. The film 100 is shaped as a meshed micro-structure obtained with segments 101, having cross section profile with nearly vertical lateral branches 102, and an upper branch 103 with substantially horizontal profile. The number 104 indicates the lower surface of the film 100. The segments 101 join in nodes 105 and delimit holes 106 with the shape of truncated cones with pentagonal plan form.

A film of this kind presents high "strike-through" and "wetback" values, which negatively influence its functionality, as well as high "gloss", which immediately reveals the nature of the material, i.e. plastic.

This prior art holed film 100, in order to reduce its superficial gloss and/or provide the rough appearance of a tissue, presents on the summit of the segments 101 or in other parts of the inner surface of the holes 106, micro-asperities or micro-projections arranged in a regular checkerboard manner, as shown in 107 in Figure 1, which, however, have the drawback of slowing the flow of the liquid, offering greater friction during its travel through the truncated cone itself. Moreover, these possible superficial projections force the fluid to remain deposited around them. The above drawbacks contribute, together with the high "wetback" value mentioned above, to the unpleasant wet sensation that results when the upper filtering layer of a baby nappy or an incontinence pad or ladies' sanitary napkin is not able to prevent liquids from returning in contact with the skin.

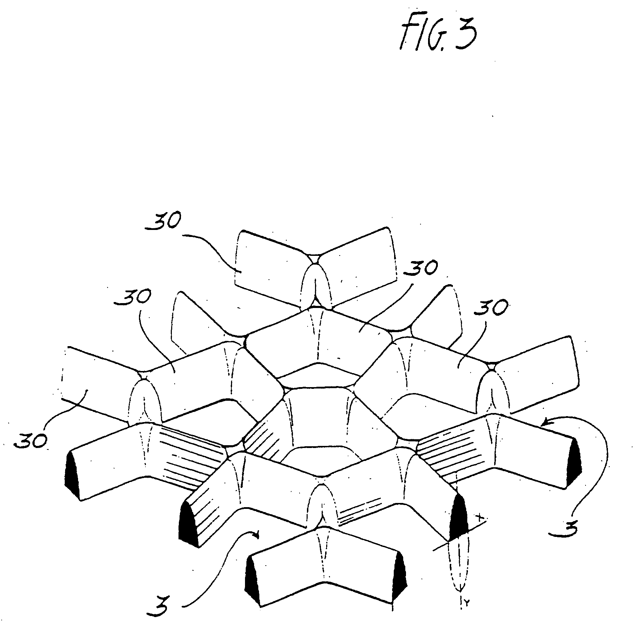

Figure 2 shows an enlarged schematic view of a second film of holed plastic material 200, known in the prior art. Unlike the matrix 100 of Figure 1, the film 200 has segments 201, having cross section profile with oblique lateral branches 202 terminating in the upper surface with a vertex 203. With this tapered configuration, "gloss" is actually reduced.

However, the goal - at times attained in an excellent manner - of making such films as similar to tissues as possible, feature that assumes particular importance if said films are to be employed for nappies and sanitary napkins, nonetheless fails to fulfil the principal purpose of these films, i.e., in the specific case of the hygienic-sanitary sector, of allowing a rapid absorption of body fluids, such as urine or menstrual liquid, from the surface of the skin. These liquids should be made to flow in the direction of an absorbing pad and they should be prevented from flowing back in the opposite direction, thereby avoiding an unpleasant wet sensation to the wearer of nappies or sanitary napkins.

Instead, especially in the case of the second film 200 of Figure 2, laboratory tests have shown that the presence of rectilinear segments in the cross section profile of the segments 201, which define conical projections, allows to provide said projections with an evident conicity, regular along the entire cross section. This cross section of regular conical shape may, however, compromise the "wetback" or the "strike-through" value, or even both. If said rectilinear segments are located in the upper part of the truncated cone of the hole or have a slight inclination with respect to the horizontal plane of the upper surface of the film, they define a truncated cone or hole that is excessively closed in the lower part. In this way the "strike-through" value is compromised, and it will be very high since the passage area of the terminal part of the truncated cone or bottom of the hole is very small. If said rectilinear segments are located in the descending lower part of the truncated cone or if they are excessively inclined, with respect to the horizontal plane of the upper surface of the film, as shown in Figure 2, they define a truncated cone with insufficiently converging walls. In this way the modest conicity of the truncated cone will cause a high "wetback" value, allowing the liquid that has just passed through to flow back in the opposite direction.

### Disclosure of Invention

Therefore, in general, the aim of the present invention is to provide a film of the type in question with the property of letting liquids be absorbed rapidly, preventing their back flow in the opposite direction.

This and other aims beside are all reached by a film made of holed plastic material shaped three-dimensionally, according to claim 1.

This profile of the film segments gives rise to a particular three-dimensional shape of the holes, akin to a funnel, which confers to the film according to the invention the property of attracting surface liquids and letting them pass rapidly only downwards, preventing them from flowing back in the opposite direction.

Unlike the known films or strips of three-dimensionally shaped holed plastic material, described above, the film according to the present invention has a very low value of "wetback", a low "strike-through" value, a low "gloss" value and a reduced surface area in contact with the user.

The film according to the present invention can be obtained using any technique known in the prior art, wherein any thermoplastic film, for instance made of polyethylene and organic and/or inorganic additives or of any mixture of polyolefin and organic and/or inorganic additives, is wrapped on a forming matrix holed or extruded at the nearly molten state directly, and otherwise, on said matrix and said film is holed for instance by means of a jet of high pressure cold or hot air or by means of a jet of high pressure cold or hot water, by means of the mechanical action of a punch or any body of any mechanical consistency of any material which partially or fully penetrates within the holes of said matrix making the film take on the shape of said forming matrix.

In particular, a matrix is provided for the realisation of the film according to the invention made in the form of a thick mesh of mutually connected elements, with conical cross section, obtained with metal deposition in successive phases.

Further features and advantages of the present invention shall be more readily apparent from the detailed description that follows of a preferred embodiment, illustrated purely by way of non limiting indication in the enclosed drawings.

### Description of the Drawings

- Figures 1 and 2 show enlarged schematic perspective views of prior art films of plastic materials;
- Figures 3 and 4 show enlarged schematic perspective views of a portion of a first embodiment of matrix and of a corresponding film of plastic material according to the present invention;
- Figure 5 shows a cross section view of a portion of the matrix of Figure 3 and a portion of the film of Figure 4 superimposed on the matrix;
- Figure 6 shows a schematic representation of the operation of the film of Figure 4;
- Figure 7 shows an enlarged perspective view of a portion of a second embodiment of film of plastic material according to the present invention:
- Figure 8 shows an enlarged schematic section of a portion of film of plastic material according to the present invention struck by light;
- Figures 9 and 10 show diagrams of the profiles of the "strike-through" and, respectively "wetback" parameters versus the pass-through area for a film according to the present invention.

### Description of the Illustrative Embodiment

With reference to Figure 3, an embodiment is shown of a matrix 3 for realising a film constituting the subject of the present invention.

Such forming matrix may be obtained by electroplating of nickel of other metals, such as copper. It is defined by a thick mesh of elements 30 mutually connected according meshes with pentagonal plan form. Each element 30 has a curved cross section having the profile of a conic section, in particular of a semi-ellipse with the lesser diameter by base according to its x-axis and with the greater semi-diameter of said semi-ellipse by height according to its y-axis.

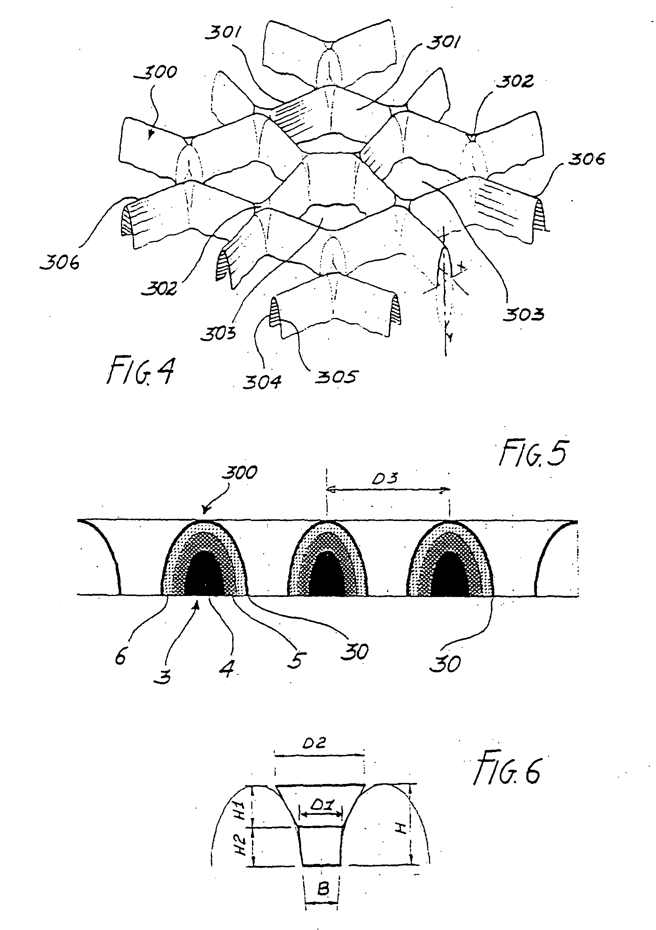

With reference to Figure 4, a film 300 is shown of holed plastic material obtained with the matrix of Figure 3. Correspondingly thereto, the film 300 according to the present invention has a micro-structure formed by segments, generically indicated with the number 310, joined in nodes, indicated as 302, and delimiting micro-holes, indicated in general as 303. Each film segment 301 presents, in cross section, a profile in the shape of a semi-ellipse, having as its vertical semi-axis the greater semi-diameter of the ellipse and as its horizontal axis the lesser diameter of the ellipse itself. In the profile of each film segment, lateral branches 304, 305 and a vertex 306 are recognised.

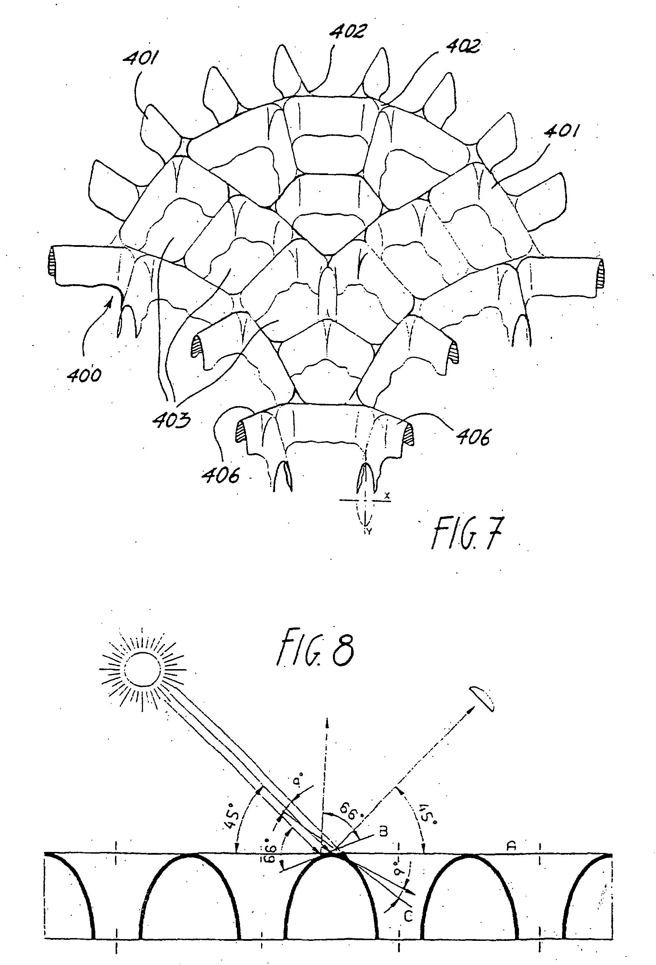

With reference to Figure 5, a film 300 is shown formed on a matrix 3, whose elements 30 are schematically represented in cross section with various layers corresponding to an equal number of phases of the electroplating process. According to a particular metal electroplating technique. a nearly vertical growth of the element can be obtained up to a certain height, and then have progressively narrowing growth as the metal is deposited, until reaching the top with a very narrow, curved upper surface. In particular, and with reference to Figure 5. this technique consists of making a first deposition to form a support base 4; with the second deposition, the second layer 5 is obtained which confers to the matrix the proper mechanical properties such as strength and tenacity. Lastly, with the third deposition an outer superficial layer 6 is obtained to create a more porous surface coating than the other layers, able to facilitate the detachment of the film after the holing phase and able to provide it with an adequate degree of surface roughness ranging from 0.1 to 6.3 µ (preferably between 0.8 and 3.2 µ).

Other construction techniques can be used to obtain such matrices, having elements with curved cross section with the profile of a conic section, although such techniques are much more costly for this purpose, such as electron discharge machining, photoengraving or any laser technique, the removal of material by means of any mechanical machining process such as punching and etching or the superposition of concentric tubular sheets previously holed with any one of said technique.

This particular shape of the matrix elements allows to describe in the matrix three-dimensional openings with a shape akin to a funnel, shown in Figure 6, rather than a shape akin to a truncated cone, as was the usual case in the prior art. With reference again to Figure 6, the funnel shape, unlike the truncated cone shape, allows to have a dual conicity, i.e. two superposed truncated cones. such as to center to the hole in the segment defined by the height H1 a mouth D2 wider than D1 and in the segment defined by the height H1 a lower truncated cone that is greater height but less pronounced conicity. The particular shape of the matrix elements, with the cross section in the shape of a semi-ellipse with its base equal to the lesser diameter and its height equal to half of the greater diameter of said semi-ellipse, confers to the film 300 segments or projections with a profile, as shown in Figure 5, which has a wide opening D3 to take in more liquid.

Furthermore, the film 300 presents better radiused walls with no sharp edges between the segment defined by the height H1 and the segment defined by the height H2, thereby facilitating the outflow of the liquid without its being minimally influenced along the entire section of the projection and, moreover, a lower truncated cone able to hinder the liquid that attempts to flow back in the opposite direction, i.e. from the lower surface of the film towards the upper one.

Figure 7 shows a second embodiment of the film, indicated as 400, wherein the disposition of the film segments, indicated generically as 401, joining into nodes 402 and delimiting holes 403, is fan-like.

The shape of the profile of the segments 401 is identical to that of the segments 301 of Figure 4. This profile of the present invention defines a summit (the vertex 304,406 of the semi-ellipse) narrowing in correspondence with the upper surface of the film, in order to define a wider opening, and a base of such a shape as to define a segment or projection with a higher truncated cone, starting from the lower surface of the film in the direction of the upper one, with the purpose of offering a greater resistance to the fluid that attempts to flow back upwards. Moreover, the contact surface on the upper part of the film defined by the film segments is reduced and akin, in theory, to a point. This characteristic thus allows to avoid residual deposits of liquid between the upper surface of the segment 301, 401 and the surface from which the liquid is to be absorbed, preventing the unpleasant sensation of wetness. The liquid just deposited on the upper.surface of the film of the present invention easily flows downward due to the curved summit of the segments 301, 401 so that it can slide either to one side or to the other of said segments. The absence of any superficial micro-projections, generally present in the films of the prior art in order to reduce their gloss and/or confer a fibre-like appearance, facilitates this down flow.

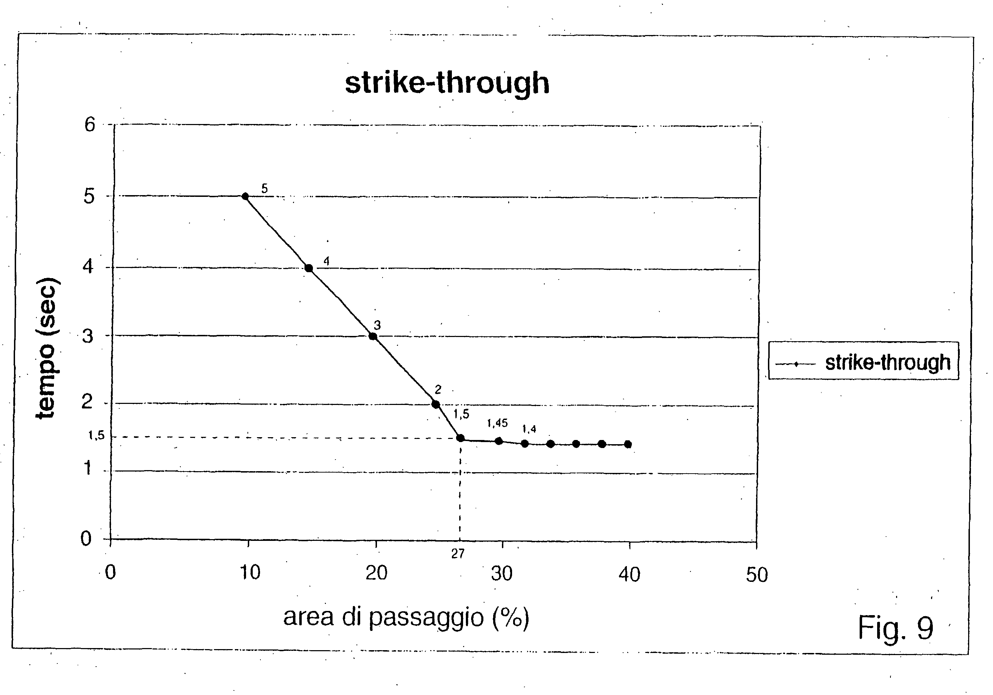

The film according to the present invention has very narrow reflecting surfaces. With reference to Figure 8, the film segment, not having rectilinear branches in the profile of its cross section and in particular on the upper surface of the film itself, offers surfaces which in theory are only point-like to incident light. This allows to obtain low values of "gloss" without having to subject the film to additional work processes, such as embossing. Considering a light beam generated by a known source positioned at a known distance which reaches the segments with an angle of incidence for instance of 45°, it can be noted that all rays which reach the upper surface A, corresponding to the upper surface of the film, nearly flat in the segment, is reflected in the direction of the receiving lens positioned at a known distance. In the case of the film according to the present invention, with the segments having a cross section of semi-elliptical shape, one can note that only a minimum portion is reflected in the direction of the receiving lens. Since the film segment has the curved shape of a semi-ellipse, the incident rays are reflected according to the tangent of each point of said curve, so that in Figure 8 it can be observed that only the central ray is reflected according to the tangent to the point on the summit of the bridge corresponding to the upper surface A of the film. The other rays have a different reflection because they have a different angle of incidence from the point of tangency. for instance of tangent B with an angle of incidence of 66°, so that, being reflected with the same angle, they are not perceived by the receiving lens. A ray with a different angle of incidence, for instance of tangent C with an angle of 9°, being reflected with the same 9° angle, is also not perceived by the receiving lens.

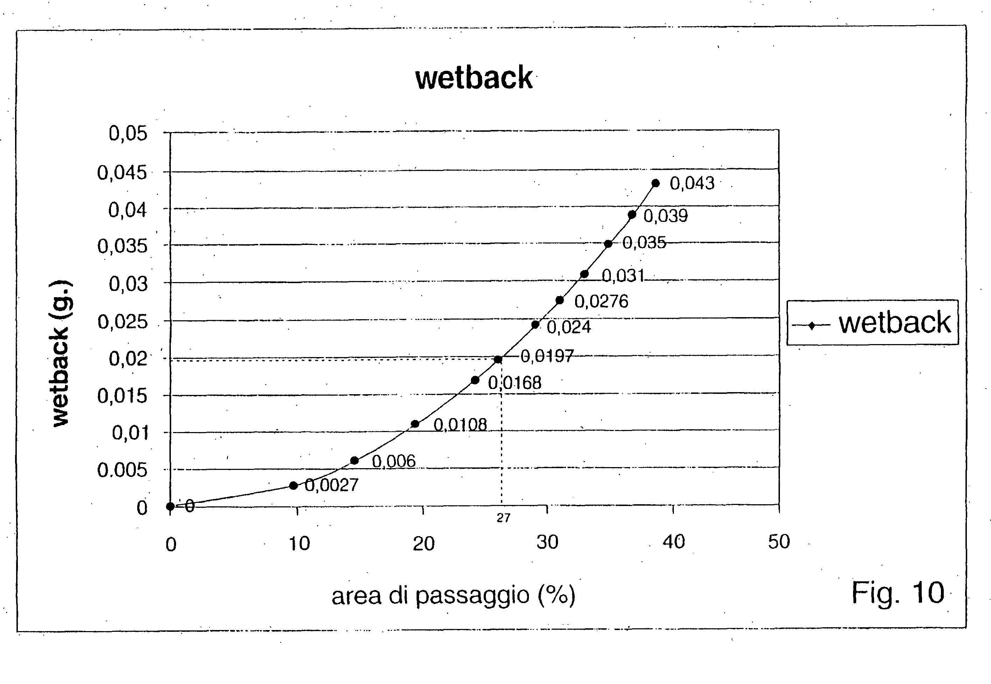

The graphs of Figures 9 and 10 show the relationship existing between "wetback" and passage area, and between "strike-through" and passage area respectively, using the film of the present invention. While the value of "wetback" has nearly regular growth as the passage area increases, the value of "strike-through" decreases regularly as the passage area increases until reaching a value of about 27%, measured at the base of the projections or film segments. The graph in Figure 9 shows that it is not necessary to increase this value any further since the value of "strike-through" remains nearly constant. This is explained because up to that value the flow of the liquid through the projections is influenced by its own surface tension and viscosity, whereas beyond that value surface tension and viscosity no longer have any influence or in any case their influence is not sufficient to slow the flow in a noticeable manner. Assuming a value of the passage area equal about 27% to be the limit beyond which it is not advantageous to proceed for the aforesaid reasons, it can be observed that it corresponds to a "strike-through" value of about 1.5 sec.

With reference to the graph in Figure 10, to a passage area of 27% corresponds a "wetback" value of about 0.02 g. Hence, it can be stated that to the value of 27% for the passage area corresponds the best trade-off between the "wetback'' and the "strike-through" values. The values reported above are in any case better than those measured in prior art films, and all trade-offs between "wetback" and "strike-through" ranging between 20 and 33% of the passage area are in any case better. This is possible thanks to the particular funnel-like shape of the film segments that have a cross section in the shape of a semi-ellipse. This shape hinders the upward back flow, opposing the liquid with a longer channel to be overcome. In the case of prior art films made of holed plastic material, instead, if the liquid flows beyond the hole at the base of the film segments, it finds less and less resistance as it flows upward. so that once it has flowed through, it is easier for the liquid to move upward than downward.

The advantages of the film of holed plastic material of the present invention, produced with matrices having a mesh of elements with curved cross section profile in the shape of a semi-ellipse can be summarised as follows:
1- very low wetback value
2- low strike-through value
3- low gloss value
4- reduced surface in contact with the user.

In conclusion the matrices with the elements defined by the present invention allow to obtain films made of holed plastic materials with better performance than those existing in the prior art or in commerce.

### Laboratory Tests

Some laboratory tests are now briefly illustrated to demonstrate the advantages of the film made of holed plastic material constituting the present invention:

The "liquid strike-through time" test consists of weighing 5 superposed ERT.FF3.W/S absorbing papers with a loading factor of 3.3, by Hollingsworth & Vose Company Ltd., stored for at least 24 hours at a humidity of 65%±2 and a temperature of 20°±2°C. The papers used for this test were subsequently used also for the wetback test. A square piece of filtering film to be tested (in our case the film of the present invention or others like it) is cut with its side measuring 125 mm and it is positioned on the upper surface of the layer of five superposed absorbing papers just weighed (in our case the holed film must have projections or segments oriented towards the absorbing papers and in contact therewith). The packet thus obtained, comprising the five absorbing papers and the filtering film, is positioned at the base of an instrument called "lister", able to meter a known quantity (5 ml) of known liquid and to measure the time taken by said liquid to flow through the filtering film being examined. The instrument used for the test is a "lister" made by Lenzing AG.

The "liquid strike-through time" test is immediately followed by the "coverstock wetback" test. The value of the weight of the five absorbing papers, previously weighed, is multiplied times the loading factor. The result of this product yields the maximum quantity of liquid that can be absorbed by said five absorbing papers in order to reach saturation. Through the "lister", used for the "liquid strike-through time" test, a further quantity of the same liquid is metered on the packet comprising the five absorbing papers and the piece of filtering film, equal to the difference between the result of the aforesaid product and the quantity of liquid previously metered in said test (5 ml). After effecting this additional metering the base contained in the "lister", together with said packet of layers of absorbing papers and filtering film, is moved into the nearby "wetback" instrument for the test by the same name, which consists of laying a known 4 kg weight on the upper surface of the filtering layer of said packet for a known time of 3 minutes with the purpose of uniformly wetting the upper surface of said filtering film. During this time, two ERT.FF3.W/S absorbing papers by Hollingsworth & Vose Company Ltd., stored for at least 24 hours at a humidity of 65%±2 and a temperature of 20°±2°C, are weighed. Once this interval has elapsed, the weight is automatically lifted and the two papers just weighed are placed on the upper surface of the same filtering film. Then the same weight is descended again for an interval of 2 minutes. Upon expiration of this interval, the two papers are weighed again. The difference between this value after the test and that prior to the test yields the weight of the liquid that has egressed from the filtering layer. The instrument used for the test is a "wetback" manufactured by Lenzing AG. The liquids used for said tests are of two kinds:
simulated urine, prepared in the laboratory by diluting 18 g. of sodium chlorine in 2 litres of distilled water in accordance with Edana 150.3, with a surface tension of 70±2nM/m and a viscosity of 1.0cPs at 20°C;
plasmion solution, produced by Bellon in April of 1997 and expiring in April 2000, Batch no. 2018-3, with a surface tension of 63±2nM/m and a viscosity of 1.6 cPs at 20°C.

The gloss tests that followed were in accordance with ASTM standards D2447 and C346 and were conducted with a micro-gloss reflectometer manufactured by BYK-Gardner GmbH.

## Claims

1. Film made of holed plastic material three-dimentionally shaped, having an upper surface presenting a multiplicity of opening extending in the form of through holes in the direction of a lower surface of the same film; mutually adjacent through holes being separated by segments of said film having a profile with symmetrical sides converging towards the upper surface, **characterised in that** said profile of the film segments has a semi-ellipse cross-section; said ellipse cross section profile having a lesser axis equal to the lesser diameter and lying on the lower surface of the film itself and with the vertex, relating to the greater axis, on the upper surface of the film itself.

2. Matrix for the realisation of the film according to Claim 1, **characterised in that** said matrix is realised in the form of a thick mesh of mutually connected elements, with conic cross section, obtained with the deposition of metal in successive phases; said each matrix elements being realised with three layers, corresponding to an equal number of phases.

## Patentansprüche

1. Folie aus perforiertem, dreidimensional geformten Plastikmaterial mit einer oberen Oberfläche, die eine Vielzahl von Öffnungen aufweist, die sich in Form von durchgehenden Bohrungen in Richtung einer unteren Oberfläche derselben Folie erstrecken; wobei die aneinandergrenzenden durchgehenden Bohrungen durch Segmente der genannten Folie voneinander getrennt sind, die ein Profil mit zur oberen Oberfläche konvergierenden symmetrischen Seiten haben, **dadurch gekennzeichnet, dass** das genannte Profil der Foliensegmente einen halbelliptischen Querschnitt hat; wobei das genannte Profil mit dem elliptischen Querschnitt eine geringere Achse hat, die gleich dem geringeren Durchmesser ist und an der unteren Oberfläche der Folie selbst liegt, und die mit der sich auf die grössere Achse beziehenden Spitze an der oberen Oberfläche der Folie selbst liegt.

2. Matrize zur Herstellung der Folie nach Patentanspruch 1, **dadurch gekennzeichnet, dass** die genannte Matrize in Form eines dichten Netzes von gegenseitig miteinander verbundenen Elementen von konischem Querschnitt hergestellt ist, erhalten durch Auftragen von Metall in aufeinanderfolgenden Phasen; wobei jedes Matrizenelement mit drei Lagen hergestellt ist, welche der gleichen Zahl von Phasen entsprechen.

## Revendications

1. Film fait d'une matière plastique percé en forme tridimensionnelle ayant une surface supérieure présentant une multiplicité d'ouvertures s'étendant en forme de trous de passage dans la direction d'une surface inférieure du même film; des trous de passage réciproquement adjacents étant séparés par des segments dudit film ayant un profil avec des côtés symétriques et convergeant vers la surface supérieure, **caractérisé en ce que** ledit profil des segments de film a en coupe la forme d'une demi-ellipse; ledit profil en ellipse en coupe ayant son petit axe égal au diamètre plus petit et disposé sur la surface inférieure du film lui-même et avec le sommet, relatif à son grand axe, sur là surface supérieure du film lui-même.

2. Matrice pour la réalisation du film selon la revendication 1, **caractérisée en ce que** ladite matrice est réalisée sous forme d'un filet à mailles serrées d'éléments reliés ensemble, de section transversale conique, obtenu par dépôt de métal en phases successives; lesdits éléments de matrice étant réalisés chacun avec trois couches correspondant à un égal nombre de phases.
